# EUROPÄISCHE PATENTANMELDUNG

(11) **EP 1 698 342 A1**
(43) Veröffentlichungstag der Anmeldung: **06.09.2006**
(21) Anmeldenummer: 06008372.2
(22) Anmeldetag: 17.12.2001
(51) Int. Cl.: A61K 31/732, A61P 31/00

(54) **Verwendung von Kohlenhydraten zur Beseitigung von Darminfektionen bei Tieren**

(30) Priorität: 31.01.2001 DE 10104055
(62) Teilanmeldung aus: 01994796.9
(71) Anmelder: Südzucker Aktiengesellschaft Mannheim/Ochsenfurt, 68165 Mannheim (DE)
(72) Erfinder: Klingeberg, Michael, 67269 Grünstadt (DE); Kozianowski, Gunhild, 67269 Grünstadt (DE); Kunz, Markwart, 67550 Worms (DE); Munir, Mohammed, 67271 Kindenheim (DE); Vogel, Manfred, 67271 Neuleiningen (DE)
(74) Vertreter: Schrell, Andreas

(57) **Zusammenfassung**

Die vorliegende Erfindung betrifft die Verwendung von Kohlenhydraten, insbesondere 1-O-α-D-Glucopyranosyl-D-sorbit, 6-O-α-D-Glucopyranosyl-sorbit, Lactobionsäure, Maltobionsäure, "condensed palatinose", Difructosedianhydriden, Fructooligosacchariden, hydrierten Fructooligosacchariden, Chitooligosacchariden, Chitosanoligosacchariden, Galactomannanoligosacchariden und Oligogalacturonid-enthaltenden Pektinhydrolysaten zur Behandlung von bakteriellen Darminfektionen bei monogastrischen Tieren sowie Tierfuttermittel und diätetische Tierfuttermittel, die eines dieser Kohlenhydrate als Zusatz enthalten.

## Beschreibung

Die vorliegende Erfindung betrifft die Verwendung von Kohlenhydraten, insbesondere 1-O-α-D-Glucopyranosyl-D-sorbit, 6-O-α-D-Glucopyranosyl-sorbit, Lactobionsäure, Maltobionsäure, "condensed palatinose", Difructosedianhydriden, Fructooligosacchariden, hydrierten Fructooligosacchariden, Chitooligosacchariden, Chitosanoligosacchariden, Galactomannanoligosacchariden und Oligogalacturonid-enthaltenden Pektinhydrolysaten zur Behandlung von bakteriellen Darminfektionen bei monogastrischen Tieren sowie Tierfuttermittel oder diätetische Tierfuttermittel, die eines dieser Kohlenhydrate als Zusatz enthalten.

Infektiöse Darmentzündungen bei Tieren können durch Bakterien, Viren, Pilze oder Parasiten verursacht werden. Das Leitsymptom solcher Darmentzündungen ist die Diarrhö. Sie können, müssen jedoch nicht mit Schleimhautveränderungen einhergehen. Das klinische Bild von infektiösen Darmentzündungen ist sehr variabel. Gleiche Infektionserreger können zu schwersten Erscheinungen wie Durchfällen, Fieber und Ernährungsstörungen führen, aber auch zu asymptomatischen Verläufen. Von Bedeutung ist ferner die Lokalisation der Infektion. Während bei Erkrankungen des oberen Intestinaltraktes Ernährungsstörungen, Meteorismus oder massige Stuhlentleerungen zu erwarten sind, gibt es bei Befall des unteren Traktes zahlreiche dünnflüssige Entleerungen. Hinzu kommen gegebenenfalls unspezifische Zeichen wie viszerale Schmerzen, Übelkeit, Erbrechen und Fieber.

Die häufigsten Erreger von Darminfektionen sind Bakterien. Voraussetzung für ihre Pathogenität ist die Fähigkeit, sich an der Darmoberfläche befestigen zu können. Beispielsweise sind nur diejenigen Kolibakterien gefährlich, die zu dieser Adhärenz befähigt sind. Einige Bakterien, wie Choleravibrionen, endotoxigene Colibakterien, Staphylococcen, Shigellen und Klebsiellen produzieren Bakterien-Toxine, die zu einer Aktivierung der intrazellulären Adenylcyclase und damit zu einer vermehrten Sekretion von Flüssigkeit und Elektrolyten führen. Derartige Bakterien-Toxine können auch zu einer direkten Zerstörung von Enterozyten führen. Einige Bakterien, wie Kolibakterien, Shigellen, Salmonellen, *Campylobacter jejuni* und *Yersinia enterocolitica* können die intestinale Epitheloberfläche durchdringen und bewirken eine Zerstörung der Schleimhaut, die an Blut- und eventuell Eiterbeimengungen zum Stuhl erkennbar wird.

Bakteriell bedingte Darminfektionen werden hauptsächlich durch Salmonellen verursacht. Aufgrund von antigenen Eigenschaften konnten etwa 1400 Salmonellen-Untertypen charakterisiert werden. Quellen sind Urin und Fäzes von erkrankten Tieren oder sogenannten asymptomatischen Dauerausscheidern, wobei die Ansteckung häufig über die Aufnahme von kontaminierten Nahrungsmitteln oder kontaminiertem Trinkwasser erfolgt. Bei Menschen gibt es beispielsweise fünf verschiedene Verlaufsformen der Salmonellen-Infektion, die im Einzelfall sehr variabel sein können. Bei 75% aller Fälle handelt es sich um akute Gastroenteritis. Bei 10% aller Fälle handelt es sich um Bakteriämie mit und ohne intestinale Begleiterscheinungen. Etwa 8% der Salmonellen-Infizierten entwickeln Typhus beziehungsweise typhoides Fieber. Bei etwa 5% der Infektionen handelt es sich um lokale Infektionen, beispielsweise der Knochen und Gelenke. Weniger als 1% aller Fälle weisen einen asymptomatischen Trägerstatus auf, wobei die Erreger länger als ein Jahr zumeist in der Gallenblase gefunden werden.

Darminfektionen mit pathogenen Keimen wie Salmonellen oder Shigellen stellen insbesondere bei der Schweinemast und der Hühnerhaltung ein gravierendes Problem dar. Dabei geht von den Tieren, die nicht selbst erkrankt sind, sondern zu den sogenannten Salmonellen-Dauerausscheidern gehören, eine besondere Gefahr für die tierische, aber auch die menschliche Gesundheit aus. So kommt es alljährlich zu einer Vielzahl von Salmonellen-Infektionen beim Menschen, die durch infizierte Fleischwaren oder andere infizierte tierische Produkte verursacht werden.

Eine Behandlung infektiöser Darmerkrankungen kann sich sowohl gegen die Folgen der Infektion, wie beispielsweise Dehydratation und Elektrolytentgleisung, als auch gegen den Erreger selbst richten. Eine gegen den Erreger gerichtete Behandlung bakterieller Darminfektionen kann beispielsweise die Verabreichung von Antibiotika umfassen. Bei bakteriellen Darminfektionen werden Antibiotika wie Ampicillin, Co-trimoxazol und Gyrasehemmer jedoch nur in schweren Fällen mit Fieber usw. empfohlen, da aus einer solchen Antibiotikabehandlung eine ungünstige Resistenzentwicklung resultieren kann. Ausnahmen bilden vor allem schwer verlaufende Shigellosen, die mit Penicillin behandelt werden können. Abgesehen von der Problematik von Antibiotika-Rückständen in Fleischwaren ist eine Antibiotika-Behandlung bei Schweinen auch und insbesondere wegen der Ausbildung von Antibiotikaresistenzen bei den zu bekämpfenden Keimen nicht mehr erwünscht. So ist über die Erkrankung eines Kindes mit Antibiotika-resistenten Salmonellen nach Kontakt mit Stalltieren berichtet worden (Food Chemical News, (Mai 2000), 21-22).

Es ist bekannt, dass in einer frühen Phase von Gastroenteritis die normale Kommensalen-Mikroflora durch Urease-produzierende pathogene Bakterien überwachsen wird (Salminen et al., Chemotherapy, 41 (Suppl.) (1995), 5-15). Eine Strategie zur Behandlung von infektiösen Darmerkrankungen verfolgt daher das Ziel, insbesondere während der Phase der akuten Diarrhö die gastrointestinale Mikroflora zu normalisieren, indem nicht-pathogene Keime enthaltende Bakterienpräparate verabreicht werden. Beispielsweise beschreibt die WO 99/26642 die Verwendung des Escherichia coli-Stammes DSM 6601 zur Behandlung mikrobiell bedingter Diarrhöen auf dem Veterinärsektor.

Ebenso ist die Verwendung von Lactulose zur Behandlung von bakteriellen Darminfektionen des Menschen bekannt (Ballongue et al., Scand. J. Gastroenterol. Suppl., 222 (1997), 41-44). Lactulose wird nicht verdaut und führt zu einem sauren Milieu im Dickdarm, wobei das Wachstum von Lactobacillus-Arten begünstigt und möglicherweise so die Elimination von Salmonellen beschleunigt wird. Es ist daher vorgeschlagen worden, Lactulose auch zur Behandlung von Salmonellen-infizierten Schweinen einzusetzen (Ley et al., Top Agrar, 10 (1999), 20-21). Bei Verwendung von Lactulose treten als Nebenwirkung jedoch erhöhte Flatulenzen und Durchfall auf, so dass die Patienten eher geneigt sind, die Einnahme von Lactulose zu verweigern. Außerdem ist. Lactulose für einen Einsatz als Futtermittel-Bestandteil zu teuer.

Ebenfalls ist die Verwendung von D-Mannose bei Hühnern in Trinkwasser bekannt (Oyofo et al., Poultry Science, 68 (1989), 1357-1360). Die so behandelten Hühner zeigten gegenüber Hühnern, die keine D-Mannose erhielten und von denen 78% bis 93% eine Salmonellenbesiedelung aufwiesen, eine wesentlich reduzierte Anfälligkeit für Salmonellenbesiedlung (21-43%). Da D-Mannose für einen allgemeinen Einsatz in Tierfutter nicht zur Verfügung steht und außerdem sehr teuer ist, wurde vorgeschlagen, lysierte und getrocknete mannosehaltige Zellwände von Hefen als Futtermittelzusatz für Hühner und Schweine einzusetzen (Bae et al., Han'guk Chuksan Hakhoechi, 41(1) (1999), 23-30). Durch eine 7-tägige Behandlung von Hühnern mit Hefezellwänden wurde die Besiedlungsdichte von Salmonella typhimurium im Caecum lediglich von 10⁵ CFU/ml auf 10⁴ CFU/ml reduziert. Dieses Ergebnis ist daher nicht befriedigend. Außerdem haben solche mannosehaltigen Substanzen den Nachteil, dass sie mit anderen Zellwandbestandteilen verunreinigt sind.

Der vorliegenden Erfindung liegt somit das technische Problem zugrunde, Mittel zur Behandlung von Darminfektionen, insbesondere bakteriell verursachten Darminfektionen, bei Tieren bereitzustellen, wobei die Mittel in höherem Maße als die bislang verwendeten Mittel eine Heilung von Darminfektionen ermöglichen, ohne dass die im Stand der Technik beschriebenen Nebenwirkungen auftreten, und wobei die Mittel gegenüber den herkömmlicherweise verwendeten Mitteln erheblich kostengünstiger, herzustellen sind.

Die vorliegende Erfindung löst dieses technische Problem durch die Verwendung mindestens eines durch die Enzyme des Verdauungstraktes nicht abbaubaren Kohlenhydrates mit Ausnahme von Lactulose, ausgewählt aus der Gruppe bestehend aus einem Oligosaccharid, einem Oligosaccharid-Derivat, einem Hydrolysat eines polymeren Kohlenhydrates und einem Gemisch davon als Wirkstoff zur Behandlung von Darminfektionen eines Tieres. Erfindungsgemäß ist vorgesehen, dass diese Kohlenhydrate insbesondere zur Behandlung von bakteriellen Darminfektionen, vorzugsweise von Darminfektionen, die von einem Mikroorganismus der Gattung *Salmonella* oder *Shigella* verursacht werden, eingesetzt werden. Die erfindungsgemäß eingesetzten Verbindungen werden von den Enzymen der Dünndarm-Mucosa nicht gespalten und gelangen somit unverändert ins Caecum und in den Dickdarm. Somit können sie ihre infektionsverhindernde beziehungsweise infektionsbeseitigende Wirkungim Gastrointestinaltrakt über einen oder mehrere der folgenden Mechanismen entfalten. Einige der erfindungsgemäß eingesetzten Verbindungen können durch anaerobe Bakterien der Darmflora zu kurzkettigen Fettsäuren (SCFA), vorzugsweise Buttersäure, fermentiert werden. Andere erfindungsgemäß eingesetzten Verbindungen können durch Bakterien der Darmflora aber auch zu Milchsäure und/oder Essigsäure fermentiert werden und dadurch eine Absenkung des pH-Wertes im Dickdarm bewirken. Durch die Absenkung des pH-Wertes kann das Wachstum nichtpathogener Bakterienarten gefördert werden, während gleichzeitig pathogene Keime in ihrem Wachstum gehemmt werden. Einige der durch die Enzyme des Verdauungstraktes nicht abbaubaren Kohlenhydrate können aber auch dadurch wirken, indem sie die Anlagerung von pathogenen Keimen an die Darm-Epithelzellen verhindern oder indem sie die Fimbrien pathogener Keime blockieren und somit deren Anheftung an die Epithelzellen verhindern. Aufgrund dieser Wirkmechanismen ist eine gezielte topische Therapie von Darminfektionen, insbesondere von bakteriellen Darminfektionen, vorzugsweise Salmonellosen und Shigellosen, bei Menschen und bei Tieren möglich.

Erfindungsgemäß ist vorgesehen, dass nicht nur ein einzelner Wirkstoff, sondern eine Kombination von Wirkstoffen zur Behandlung von Darminfektionen, insbesondere von Salmonellosen und/oder Shigellosen, verwendet werden kann. Besonders vorteilhaft: ist, dass diese Wirkstoffe je nach Verlaufsform einer Darminfektion entweder in Form einer pharmazeutischen Zusammensetzung oder als Tierfuttermittelzusatz oder Trinkwasserzusatz an ein erkranktes Tier verabreicht werden können. Erfindungsgemäß ist insbesondere vorgesehen, dass bei schweren akuten Darminfektionsverlaufsformen die Wirkstoffe, einzeln oder in Kombination, in relativ hoher Konzentration in Form einer pharmazeutischen Zusammensetzung verabreicht werden. Bei milderen Verlaufsformen oder zur Prophylaxe erfolgt die Zufuhr der erfindungsgemäß verwendeten Wirkstoffe erfindungsgemäß dadurch, dass die Wirkstoffe als Zusatz in Tierfutter oder in Trinkwasser enthalten sind und somit mit der Nahrung in den Darm gelangen können. Ein besonderer Vorteil besteht auch darin, dass die erfindungsgemäßen Wirkstoffe mittels bekannter Verfahren in großen Mengen einfach und kostengünstig hergestellt werden können.

Da die Wirkstoffe im Magen und im Dünndarmbereich des Verdauungstraktes nicht abgebaut werden, sondern erst im Dickdarm, ist die Bioverfügbarkeit der erfindungsgemäßen Wirkstoffe am Wirkort außerordentlich hoch. Im Gegensatz zu herkömmlichen Wirkstoffen wie Antibiotika spielen pharmazeutische und physiologische Faktoren, die bei herkömmlichen Arzneimitteln die Bioverfügbarkeit eines Wirkstoffes in erheblichem Maße beeinflussen können, keine Rolle. Auch die präsystemische Elimination (first pass effect), das heißt der metabolische Abbau von Wirkstoffen, bevor sie an ihren Wirkort gelangen, der ansonsten die Bioverfügbarkeit von Wirkstoffen erheblich einschränken kann, spielt somit keine nennenswerte Rolle.

Vorzugsweise handelt es sich bei dem zu behandelnden Organismus um ein monogastrisches Tier. Im Zusammenhang mit der vorliegenden Erfindung werden unter dem Begriff "monogastrische Tiere" Wirbeltiere, insbesondere Vögel und Säugetiere, verstanden, bei denen der Magen hauptsächlich aus einem Muskelmagen besteht. Bei den prinzipiell monogastrischen, Vögeln kann der Muskelmagen einen als Drüsenmagen bezeichneten Abschnitt besitzen. Bei monogastrischen Säugetieren, insbesondere carnivoren und omnivoren Typen, umfasst der Magen drei Hauptabschnitte, die aus der Cardia in der Nähe der Mündung des Ösophagus, dem Fundus, das heißt dem Hauptteil des Magens, mit tubulären Fundusdrüsen und dem Pylorus in der Nähe des Magenausgangs mit verzweigten mucösen Drüsen bestehen. Der Begriff "monogastrische Tiere" schließt daher Wiederkäuer aus, bei denen der Magen vier aufeinanderfolgende anatomisch unterscheidbare Abschnitte umfaßt, nämlich den Pansen, in dem der chemische Aufschluss der Nahrung, insbesondere die Cellulose-Verdauung, durch symbiontische Mikroorganismen erfolgt, den Netzmagen beziehungsweise die Haube, den Blättermagen und den Labmagen. Im Gegensatz zu monogastrischen Tieren wird bei Wiederkäuern die Nahrung von der Haube zunächst in die Mundhöhle zurücktransportiert. Nach dem Wiederkäuen gelangt die Nahrung über die Haubenrinne in den Blättermagen, von dem aus die Flüssigkeiten und der eingedickte Nahrungsbrei in den Labmagen transportiert werden.

Bei monogastrischen Tieren handelt es sich insbesondere um Nutztiere, die keine Wiederkäuer sind und vom Menschen zu Zwecken der Arbeitsleistung, des Nahrungsmittelbedarfs oder zur Gewinnung von Rohstoffen in landwirtschaftlichen Betrieben gehalten werden. Vorzugsweise handelt es sich bei solchen Nutztieren um Tiere, die entweder selbst von infektiösen Darmerkrankungen, beispielsweise durch Salmonellen oder Shigellen verursachten Darminfektionen, betroffen sind oder die Dauerausscheider für derartige infektiöse Keime sind, wobei die Keime erst später bei anderen Organismen, beispielsweise beim Menschen, über die Nahrungsmittelkette zu Infektionen führen können. Vorzugsweise handelt es sich bei solchen Nutztieren um Schweine, Gänse, Hühner, Enten und Puten.

Im Zusammenhang mit der vorliegenden Erfindung werden unter dem Begriff "Darminfektionen" oder "infektiöse Darmentzündungen" solche Erkrankungen des Darmes verstanden, die als Folge einer Besiedelung mit Bakterien, Viren, Pilzen oder Parasiten entstehen. Der Begriff umfasst insbesondere bakteriell bedingte Infektionen des Darmes wie die verschiedenen Salmonellose-Verlaufsformen, insbesondere Gastroenteritis, Bakteriämie, Typhus beziehungsweise typhoides. Fieber, lokale Salmonelleninfektionen und/oder den asymptomatischen Trägerstatus, Dysenterie, insbesondere Shigellose, Campylobacter-Enteritis und Yersiniosen, hämorrhagische Colitis, beispielsweise verursacht durch E. coli O157, sowie bakteriell-toxische Erkrankungen wie Staphylococcen-Enteritis. Der Begriff umfasst ebenfalls alle extraintestinalen Begleiterkrankungen infektiöser Darmentzündungen, beispielsweise Organinfektionen, die häufig bei Salmonellen-Bakteriämie auftreten, wie Pneumonie, Arthritis, Cholecystitis und ähnliche.

Im Zusammenhang mit der vorliegenden Erfindung werden unter einem "Wirkstoff" jedwede Stoffe, insbesondere Stoffe chemischer Natur verstanden, welche biologische Zellen oder Teile davon, insbesondere Zellorganellen oder Zellbestandteile, beeinflussen oder erkennen können. Im Zusammenhang mit der vor liegenden Erfindung werden unter dem Begriff "Wirkstoffe" insbesondere Therapeutika verstanden, also Stoffe, die entweder prophylaktisch oder krankheitsbegleitend eingesetzt werden können, um Krankheitszustände, insbesondere infektiöse Darmentzündungen, zu vermeiden, zu lindern und/oder zu beseitigen.

Unter "durch die Enzyme des Verdauungstraktes nicht abbaubaren Kohlenhydraten" werden insbesondere Polyhydroxyaldehyde und Polyhydroxyketone sowie höhermolekulare Verbindungen, die sich durch Hydrolyse in Polyhydroxyaldehyde und Polyhydroxyketone überführen lassen, verstanden, die bei der Verdauung, das heißt nach mechanischer Zerkleinerung von Nahrungsstoffen, deren Verflüssigung durch Speichel und Ansäuerung durch den Magensaft, durch die Verdauungsenzyme im Dünndarmbereich des Verdauungstraktes nicht in resorptionsfähige Bestandteile abgebaut und somit nicht in das Blut beziehungsweise die Lymphe aufgenommen werden können. Die unverdauten beziehungsweise unverdaubaren Kohlenhydrate können allenfalls im Dickdarm eines monogastrischen Tieres durch die Bakterienflora in niedermolekulare Bestandteile abgebaut werden. Bei den im Dünndarm eines monogastrischen Tieres nicht abbaubaren Kohlenhydraten handelt es sich vorzugsweise um Oligosaccharide, Derivate davon oder Hydrolysate von polymeren Kohlenhydraten.

Im Zusammenhang mit der vorliegenden Erfindung wird unter einem "Oligosaccharid" eine Verbindung verstanden, die durch Kondensation von 2 bis 10 Monosacchariden entsteht. Vorzugsweise handelt es sich dabei um ein Disaccharid, ein Trisaccharid oder ein Tetrasaccharid mit Ausnahme von Lactulose. Im Zusammenhang mit der vorliegenden Erfindung werden unter "Derivaten" funktionelle Äquivalente oder Abkömmlinge eines erfindungsgemäß verwendbaren Oligosaccharides verstanden, die unter Beibehaltung der-Grundstruktur dieses Oligosaccharides durch Substitution von Atomen oder Molekülgruppen beziehungsweise -resten erhalten werden und/oder deren chemischer Aufbau sich von dem des Oligosaccharides an mindestens einer Position unterscheidet. Die Unterschiede zwischen einem Derivat und einem Oligosaccharid können beispielsweise durch Additionsreaktionen, Eliminierungsreaktionen oder Substitutionsreaktionen entstanden sein. Solche Derivate können durch Isolierung aus natürlichen Rohstoffen gewonnen oder durch Abwandlung bereits vorhandener Verbindungen oder gegebenenfalls durch Totalsynthese hergestellt werden.

Im Zusammenhang mit der vorliegenden Erfindung werden unter einem "Hydrolysat eines polymeren Kohlenhydrates" Polysaccharide, Polyuronide, Mucopolysaccharide, Glycoproteine, Glycolipide, Mureine und Kapsel- und Schleimsubstanzen von Polysaccharidnatur verstanden, die unter Einwirkung von Wasser gespalten wurden. Eine derartige Hydrolyse kann entweder mittels entsprechender chemischer Reaktionen oder unter Verwendung von Enzymen, beispielsweise Hydrolasen, wie Pektinlyase, erfolgen.

In einer bevorzugten Ausführungsform der Erfindung wird ein Disaccharid, Trisaccharid, Tetrasaccharid, ein Oxidationsprodukt davon, ein Reduktionsprodukt davon, ein Kondensationsprodukt davon oder ein Anhydrid dieser Verbindungen als Wirkstoff zur Behandlung von entzündlichen Darminfektionen verwendet. Unter einem "Oxidationsprodukt" wird ein Produkt verstanden, das erhalten wird, wenn der zu oxidierende Stoff Elektronen an ein Oxidationsmittel abgibt. Oxidationsprodukte von Zuckern wie Oligosacchariden sind beispielsweise Onsäuren, Uronsäuren und Zuckerdicarbonsäuren. Unter "Reduktionsprodukten" werden Produkte verstanden, die durch chemische Abspaltung von Sauerstoff aus einer Verbindung durch ein oder mehrere Desoxidationsmittel erhalten werden. Ein Merkmal des Reduktions- beziehungsweise Hydrierungsvorganges besteht darin, dass der zu reduzierende Stoff Elektronen von dem Reduktionsmittel aufnimmt. Reduktionsprodukte von Oligosacchariden sind beispielsweise aliphatische Zuckeralkohole und Cyclitole oder Cyclite. Im Zusammenhang mit der vorliegenden Erfindung wird unter einem "Kondensationsprodukt" ein Produkt verstanden, das bei einer unter katalytischem Einfluss verlaufenden chemischen Reaktion erhalten wird, wobei sich mindestens zwei Moleküle unter Abspaltung eines einfachen Moleküles wie Wasser zu einem größeren Molekül vereinigen. Unter Dianhydriden werden Verbindungen verstanden, die durch Abspaltung von zwei Wassermolekülen durch Erhitzen entstehen.

In einer besonders bevorzugten Ausführungsform werden 1-O-α-D-Glucopyranosyl-D-sorbit (im Folgenden 1,1-GPS) und/oder 6-O-α-D-Glucopyranosyl-D-sorbit (im Folgenden 1,6-GPS) als Wirkstoff zur Behandlung bakteriell bedingter Darminfektionen verwendet.

Verfahren zur Herstellung der Zuckeralkohole 1,1-GPS und 1,6-GPS beziehungsweise diese enthaltende Gemische sind bekannt. In der DE 195 23 008 A1 wird beispielsweise ein Verfahren zur Herstellung von Gemischen aus 1,6-GPS und 1-O-α-D-Glucopyranosyl-D-mannit (1,1-GPM) offenbart, dass die Hydrierung von Isomaltulose bei Drücken unter 50 Atmosphären unter Verwendung von Katalysatoren umfasst. In der EP 0 625 578 B1 werden Verfahren zur Gewinnung von 1,1-GPM, 1,1-GPS und 1,6-GPS enthaltenden Zuckeralkoholgemischen beschrieben, bei dem zunächst Saccharose in ein Isomaltulose- und Trehalulose-haltiges Gemisch umgewandelt und das so erhaltene Produkt katalytisch zu einem 1,1-GPM, 1,1-GPS und 1,6-GPS enthaltenden Gemisch hydriert wird.

In einer besonders bevorzugten Ausführungsform der Erfindung wird ein Disaccharidalkohol-Gemisch eingesetzt, das 1-0-α-D-Glucopyranosyl-D-mannit (1,1-GPM) und 6-O-α-D-Glucopyranosyl-D-sorbit (1,6-GPS) enthält, wobei in besonders bevorzugter Ausführungsform die vorgenannten beiden Disaccharidalkohole in Mengenverhältnissen von 1:99 bis 99:1, vorzugsweise etwa 50:50 (Gew.-%), eingesetzt werden. Die nahezu äquimolare Mischung von 1,1-GPM und 1,6-GPS ist im Handel erhältlich und wird als Palatinit® oder Isomalt bezeichnet. Selbstverständlich können auch 1,1-GPM- oder 1,6-GPS-angereicherte Gemische verwendet werden, wie sie in der DE 195 32 396 C2 beschrieben sind.

In einer weiteren bevorzugten Ausführungsform wird ein Disaccharidalkohol-Gemisch eingesetzt, dass die Komponenten 1,1-GPM, 1,6-GPS und 1,1-GPS enthält, gegebenenfalls in Verbindung mit Mannit, Sorbit und Oligomeren. Ein derartiges Gemisch ist in der EP 0 625 578 B1 beschrieben. Der Offenbarungsgehalt der vorgenannten Druckschriften ist hinsichtlich der Herstellung und Zusammensetzung der Zuckergemische vollständig in den Offenbarungsgehalt der vorliegenden Lehre einbezogen und es wird dafür im Zusammenhang mit der vorliegenden Lehre Schutz tibegehrt.

In einer weiteren besonders bevorzugten Ausführungsform werden Lactobionsäure, Maltobionsäure, "condensed palatinose", das heißt das bei der Kondensation von Isomaltulosemolekülen entstehende Produkt, und/oder ein Difructosedianhydrid als Wirkstoffe zur Behandlung bakteriell bedingter Darminfektionen verwendet.

Lactobionsäure und Maltobionsäure lassen sich durch Oxidation von Lactose beziehungsweise Maltose herstellen. Difructosedianhydride lassen sich beispielsweise enzymatisch beziehungsweise chemisch durch Erhitzen aus Inulin und/oder Fructooligosacchariden herstellen. So können Fructooligosaccharide mittels der Inulinfructotransferase von *Arthrobacter ureafaciens* in Difructosedianhydrid III umgewandelt werden.

In einer weiteren bevorzugten Ausführungsform werden ein Fructooligosaccharid, ein hydriertes Fructooligosaccharid, ein Chitooligosaccharid, ein Chitosanoligosaccharid und/oder ein Galactomannanoligosaccharid als Wirkstoff zur Behandlung bakterieller Darminfektionen verwendet.

Im Zusammenhang mit der vorliegenden Erfindung werden unter "Fructooligosacchariden" Saccharide verstanden, die aus 2 bis 10 Fructoseeinheiten bestehen, die β-glycosidisch miteinander verbunden sind. Fructooligosaccharide können beispielsweise unter Verwendung von Endo-Inulinase aus Inulin hergestellt werden. Unter "hydrierten Fructooligosacchariden" werden Fructooligosaccharide verstanden, bei denen unter Verwendung von Katalysatoren und/oder Anwendung höherer Drücke Wasserstoff-Reste angelagert wurden. Im Zusammenhang mit der vorliegenden Erfindung werden unter "Chitooligosacchariden" Aminozucker-haltige Saccharide verstanden, die aus 2 bis 10 N-Acetyl-D-glucosamin-Resten bestehen, die β-1,4-glycosidisch miteinander verbunden sind. Unter "Chitosanoligosacchariden" werden deacetylierte Chitooligosaccharide verstanden. Unter "Galactomannanoligosacchariden" werden Saccharide verstanden, bei denen die Ketten aus zwei bis zehn 1->4 verknüpften Mannose-Einheiten bestehen, die in der β-Pyranose-Form vorliegen und an die Galactose-Moleküle als kurze 1->6-Verzweigungen geknüpft sind.

In einer weiteren bevorzugten Ausführungsform wird ein Oligogalacturonid-enthaltendes Pektinhydrolysat als Wirkstoff zur Behandlung bakterieller Darminfektionen verwendet.

Erfindungsgemäß ist insbesondere vorgesehen, dass bei Vorliegen einer Darminfektion, vorzugsweise einer bakteriellen Darminfektion wie einer Salmonellose oder Shigellose, der Wirkstoff an ein monogastrisches Tier in einer Dosis verabreicht wird, die ausreicht, den Zustand der Darminfektion zu heilen oder ihm vorzubeugen, die Progression der Darminfektion zu stoppen und/oder die Symptome der Darminfektion zu lindern. Die Dosierung des Wirkstoffes erfolgt daher so, dass ein optimaler arzneitherapeutischer Effekt ohne wesentliche toxische Nebenwirkungen erreicht wird, wobei der Behandlungserfolg langfristig andauert. Die Menge des an ein Tier zu verabreichenden Wirkstoffes hängt unter anderem von der Darreichungsform, dem Alter, dem Geschlecht und dem Körpergewicht des zu behandelnden Organismus und der Schwere der Erkrankung ab. Die genaue Dosis, mit der ein monogastrisches Tier zu behandeln ist, muss daher von dem behandelnden Tierarzt individuell festgelegt werden.

In einer bevorzugten Ausführungsform der Erfindung ist daher vorgesehen, dass die Verwendung eines durch die Enzyme des Verdauungstraktes nicht abbaubaren Kohlenhydrates als Wirkstoff zur Behandlung von Darminfektionen, insbesondere von Salmonellosen oder Shigellosen, dadurch erfolgt, dass der isolierte und gereinigte Wirkstoff in Form einer pharmazeutischen Zusammensetzung an ein monogastrisches Tier verabreicht wird. Eine Verabreichung der erfindungsgemäßen Wirkstoffe in pharmazeutischen Zusammensetzungen ist insbesondere bei schweren akuten Verlaufsformen von bakteriellen Darminfektionen indiziert. Unter einer "pharmazeutischen Zusammensetzung" wird im Zusammenhang mit der vorliegenden Erfindung ein zu diagnostischen, therapeutischen und/oder prophylaktischen Zwecken verwendetes, natürliche oder synthetisch hergestellte Wirkstoffe umfassendes Gemisch verstanden, wobei die pharmazeutische Zusammensetzung die Wirkstoffe in einer beim Tier gut applizierbaren Form enthält. Die pharmazeutische Zusammensetzung kann sowohl ein festes als auch ein flüssiges Gemisch sein. Beispielsweise kann eine den Wirkstoff umfassende. pharmazeutische Zusammensetzung einen oder mehrere pharmazeutisch verträgliche Excipienten enthalten. Darüber hinaus kann die pharmazeutische Zusammensetzung üblicherweise auf dem Fachgebiet verwendete Zusatzstoffe, wie Stabilisatoren, Verdickungsmittel, Trennmittel, Sprengmittel, Gleitmittel, Farbstoffe, Geruchsstoffe, Geschmacksstoffe, Emulgatoren oder andere üblicherweise verwendete Stoffe umfassen.

Erfindungsgemäß ist vorgesehen, dass die den Wirkstoff enthaltende pharmazeutische Zusammensetzung vorzugsweise oral verabreicht wird. Eine orale Verabreichung des Wirkstoffes ist insbesondere deshalb bevorzugt, weil die zu behandelnden Erkrankungen Teile des Verdauungstraktes betreffen und die Wirkstoffe somit den betroffenen Organen direkt zugeführt werden können. Erfindungsgemäß wird insbesondere bevorzugt, dass der Wirkstoff in Form einer Suspension, Tablette, Pille, Kapsel, eines Granulates, Pulvers oder einer ähnlich geeigneten Darreichungsform verabreicht wird. Obwohl die erfindungsgemäß verwendeten Wirkstoffe gegenüber Magensäure relativ unempfindlich sind, sind Arzneimittelformen bevorzugt, die eine Magensaft-resistente Beschichtung aufweisen, so dass die in der pharmazeutischen Zusammensetzung enthaltenen Wirkstoffe den Magen ungehindert passieren können und vorzugsweise erst in den oberen Darmabschnitten in Lösung gehen. Die Zusammensetzung von Magensaft-resistenten Beschichtungen und Verfahren für die Herstellung solcher Magensaft-resistenter Beschichtungen sind auf dem Fachgebiet bekannt. In einer besonders bevorzugten Ausführungsform der Erfindung werden Arzneimittelformen verwendet, die einen verzögerten Wirkstoff-Freisetzungsmechanismus aufweisen, um somit eine längerfristige topische Therapie von bakteriellen Darminfektionen zu ermöglichen. Der Aufbau und die Zusammensetzung solcher Arzneimittelformen mit verzögerter Wirkstoff-Freisetzung sind ebenfalls auf dem Fachgebiet bekannt.

Eine Verabreichung der erfindungsgemäßen Wirkstoffe in pharmazeutischen Zusammensetzungen ist insbesondere bei akuten Verlaufsformen von entzündlichen Darminfektionen indiziert. Dies ist beispielsweise der Fall, wenn es zu schweren Durchfällen und einer damit einhergehenden verstärkten Sekretion von Flüssigkeit und Elektrolyten kommt, deren bedrohliche Folgen Austrocknung, Schock und Azidose sein können. Insbesondere bei solchen schweren akuten Verlaufsformen kann eine Kombinationstherapie: durchgeführt werden, wobei gleichzeitig mit dem/den erfindungsgemäß verwendeten Wirkstoff(en) mindestens ein weiteres Arzneimittel für die gleiche Indikation verabreicht wird. Der/die erfindungsgemä-βe (n) Wirkstoff(e) und das mindestens eine zusätzliche Arzneimittel können entweder getrennt oder in Form fixer Kombinationen verabreicht werden. Die kombinierte Anwendung des/der erfindungsgemäßen Wirkstoffs/Wirkstoffe und des mindestens einen zusätzlichen Arzneimittels kann auf die Verstärkung von therapeutischen Wirkungen abzielen, kann jedoch auch auf verschiedene biologische Systeme im Organismus wirken und so die Gesamtwirkung verstärken. Beispielsweise können der/die erfindungsgemäß verwendete(n) Wirkstoff(e) zusammen mit speziell abgestimmten Elektrolyt-Zucker-Lösungen, wie beispielsweise Elotrans®, kombiniert werden, die ebenfalls oral verabreicht werden können. In Ausnahmefällen, das heißt insbesondere bei lebensbedrohlichen akuten Verlaufsformen, können die erfindungsgemäß verwendeten Wirkstoffe ebenfalls mit Antibiotika, wie beispielsweise Ampicillin, Chloramphenicol, Tetracyclinen und ähnlichem kombiniert werden.

In einer weiteren bevorzugten Ausführungsform der Erfindung ist vorgesehen, dass die Verwendung der durch die Enzyme des Verdauungstraktes nicht abbaubaren Kohlenhydrate zur Behandlung von Darminfektionen dadurch erfolgt, indem diese Kohlenhydrate als Zusatz in Tierfuttermitteln, diätetischen Tierfuttermitteln oder in Trinkwasser enthalten sind und somit mit der aufgenommenen Nahrung in den Verdaungstrakt gelangen. Die Zufuhr der erfindungsgemäß verwendeten Kohlenhydrate über die Nahrung ist erfindungsgemäß insbesondere bei milden Verlaufsformen bakterieller Darminfektionen vorgesehen. Bei regelmäßiger Verfütterung von beispielsweise Tierfuttermitteln, die solche Kohlenhydrate enthalten, ist außerdem eine langfristige Prophylaxe bakterieller Darminfektionen möglich.

Im Zusammenhang mit der vorliegenden Erfindung werden unter "Futtermitteln" oder "Tierfuttermitteln" jedwede Stoffe oder Stoffgemische verstanden, die dazu bestimmt sind, in unverändertem, zubereitetem, bearbeitetem oder verarbeitetem Zustand an Tiere verfüttert zu werden. Tierfuttermittel können sowohl in fester Form als auch in flüssiger Form vorliegen. Die Begriffe "Futtermittel" und "Tierfuttermittel" umfassen daher auch Trinkwasser für Tiere. Bei den Futtermitteln kann es sich sowohl um Einzelfuttermittel als auch um Mischfuttermittel handeln. Die erfindungemäßen Wirkstoffe können dem Tierfutter sowohl in gelöster Form als auch in festem Zustand beigemengt werden. Zur Verabreichung an Schweine können die erfindungsgemäßen Wirkstoffe beispielsweise in Pulverform den zur tierischen Ernährung verwendeten Mineralstoffgemischen beigemengt werden. Zur Verabreichung an Hühner können die erfindungsgemäß verwendeten Wirkstoffe beispielsweise in pulverförmiger Form dem Eierlegepulver beigegeben werden.

Im Zusammenhang mit der vorliegenden Erfindung werden unter "diätetischen Tierfuttermitteln" Tierfuttermittel verstanden, die dazu bestimmt sind, einem bestimmten Ernährungszweck dadurch zu dienen, dass sie die Zufuhr bestimmter Nährstoffe oder anderer ernährungsphysiologisch wirkender Stoffe in einem bestimmten Mengenverhältnis oder in einer bestimmten Beschaffenheit bewirken. Diätetische Tierfuttermittel unterscheiden sich somit durch ihre Zusammensetzung oder ihre Eigenschaften maßgeblich von Tierfuttermitteln vergleichbarer Art. Diätetische Tierfuttermittel dienen häufig einem besonderen Ernährungszweck, insbesondere indem sie dazu beitragen, bestimmten Ernährungsanforderungen, wie sie beispielsweise aufgrund von Krankheiten, Funktionsanomalien oder allergischen Reaktionen gegen einzelne Tierfuttermittel beziehungsweise Tierfuttermittel-Inhaltsstoffe vorliegen, zu entsprechen. Diätetische Tierfuttermittel können ebenfalls sowohl in fester Form als auch in flüssiger Form vorliegen.

Die erfindungsgemäß verwendeten Wirkstoffe können ebenfalls dem Trinkwasser für Tiere zugegeben werden. Der Zusatz der erfindungsgemäßen Wirkstoffe zu Trinkwasser erfolgt vorzugsweise unmittelbar vor Gebrauch, indem die erfindungsgemäß verwendeten Wirkstoffe dem Trinkwasser beispielsweise in Form von Pulvern oder Granulaten zugesetzt werden, so dass die Wirkstoffe vorzugsweise rasch und rückstandslos in Lösung gehen können.

Die vorliegende Erfindung betrifft auch Tierfuttermittel und diätetische Tierfuttermittel, die 1-O-*α-*D-Glucopyranosyl-D-sorbit, 6-O-α-D-Glucopyranosyl-D-sorbit, Lactobionsäure, Maltobionsäure, "condensed palatinose", ein Difructosedianhydrid, ein Fructooligosaccharid, hydriertes Fructooligosaccharid, Chitooligosaccharid, Chitosanoligosaccharid, Galactomannanoligosaccharid und/oder ein Oligogalacturonid-enthaltendes Pektinhydrolysat als Zusatz enthalten. Solche erfindungsgemäßen Tierfuttermittel bewirken bei regelmäßiger Verfütterung an monogastrische Tiere einen langfristigen Schutz vor bakteriellen Darminfektionen, insbesondere durch Bakterien der Gattung *Salmonella, Shigella* oder *E.coli* verursachten Darminfektionen.

Die Erfindung betrifft auch die vorgenannten Verwendungen der; vorgenannten Kohlenhydrate und Derivate davon, wobei diese Kohlenhydrate und/oder Derivate davon für die Herstellung von pharmazeutischen Präparaten für die Therapie, also Prophylaxe und Heilung, der genannten Krankheitsbilder verwendet werden.

Weitere vorteilhafte Ausgestaltungen der Erfindung ergeben sich aus den Unteransprüchen.

Die Erfindung wird anhand der folgenden Beispielen näher erläutert.

### Beispiel 1: Herstellung von Kohlenhydrat-Verbindungen

### 1,1-GPS

Trehalulose, hergestellt gemäß DE 32 41 788, wird in entsalztem Wasser so gelöst, dass eine 40%-ige (w/w) Lösung erhalten wird, und bei 120°C und 10 Mpa Wasserstoffdruck in Gegenwart eines Raney-Nickel-Katalysators hydriert. Aus dem entstehenden Gemisch aus 1,1-GPS und 1-O-α-D-Glucopyranosyl-D-mannit (1,1-GPM) wird ein Teil des 1,1-GPM durch Kristallisation entfernt. Das im restlichen Gemisch verbleibende 1,1-GPM stört beim erfindungsgemäßen Einsatz des 1,1-GPS nicht. Deshalb kann das restliche Gemisch aus 1,1-GPS und 1,1-GPM ohne weitere Aufreinigung in Futtermitteln eingesetzt werden.

### 1, 6-GPS

Isomaltulose, hergestellt gemäß EP 28 900, wird in entsalztem Wasser so gelöst, dass eine 40%-ige (w/w) Lösung erhalten wird, und bei 120°C und 10 Mpa Wasserstoffdruck in Gegenwart eines Raney-Nickel Katalysators hydriert. Aus dem entstehenden Gemisch aus 1,6-GPS und 1-O-α-D-Glucopyranosyl-D-mannit (1,1-GPM) wird ein Teil des 1,1-GPM durch Kristallisation entfernt. Das im restlichen Gemisch verbleibende 1,1-GPM stört beim erfindungsgemäßen Einsatz des 1,6-GPS nicht. Deshalb kann das restliche Gemisch aus 1,6-GPS und 1,1-GPM ohne weitere Aufreinigung in Futtermitteln eingesetzt werden.

### Lactobionsäure

In einer kontinuierlich arbeitenden Oxidationsanlage werden 80 g Lactose-Monohydrat in entsalztem Wasser so gelöst, dass eine 4%-ige Lösung erhalten wird. Die Lösung wird dann mit Sauerstoff gesättigt. Die Oxidation erfolgt an einem Pt/C-Festbettkatalysator bei 40°C. Das entstehende Oxidationsprodukt wird kontinuierlich über eine Elektrodialyse-Einheit abgetrennt. Nach Gefriertrocknung wird Lactobionsäure (Na-Salz) als Feststoff mit einer Reinheit von mindestens 86% isoliert.

### Maltobionsäure

In einer kontinuierlich arbeitenden Oxidationsanlage werden 100 g Maltose-Monohydrat in entsalztem Wasser so gelöst, dass eine 5%-ige Lösung erhalten wird. Dann wird die Lösung mit Sauerstoff gesättigt. Die Oxidation erfolgt an einem Pt/C-Festbettkatalysator bei 40°C. Das entstehende Oxidationsprodukt wird kontinuierlich über eine Elektrodialyse-Einheit abgetrennt. Nach Gefriertrocknung wird Maltobionsäure (Na-Salz) als Feststoff mit einer Reinheit von mindestens 85% isoliert.

### Condensed Palatinose

0,15 kg gemahlene Isomaltulose werden nach Zugabe von gemahlener Zitronensäure (0,15 g) auf 160°C erhitzt und bei dieser Temperatur für 60 Minuten belassen. Die Schmelze wird mit VE-Wasser auf 45% Trockensubstanz verdünnt und am Ca²⁺-beladenen Kationenaustauscher (IMAC-Ca²⁺, Fa. Rohm & Haas) (20 1-Säulenbett) bei 70°C mit Wasser als Eluent chromatographiert. Fraktionen, die den höhermolekularen Anteil (≥ DP 4) enthalten, werden vereint, eingedampft und sprühgetrocknet. Tabelle 1 zeigt die Zusammensetzung von "condensed palatinose" nach gelchromatographischer Auftrennung.

**Tabelle 1 Zusammensetzung von "condensed palatinose"**

| | % Fläche |
|---|---|
| DP 1 | 1 |
| DP 2 | 13 |
| > DP 2 | 86 |

### Difructosedianhydrid I und II (DFA I und DFA II)

40 g Inulin (Raftiline® HP) wurden in einem zylindrischen Stahlgefäß 40 Minuten bei 200°C in einem Ölbad erhitzt. Die Difructosedianhydride wurden nach Abkühlen aus der Schmelze extrahiert (3 x 200 ml Methanol). Die vereinigten Extrakte wurden im Rotationsverdampfer bis zur Trockne eingeengt und anschließend wieder in VE-Wasser gelöst. Die 10% Trockensubstanz enthaltende Lösung (100 ml) wurde mittels Gelpermeations-Chromatographie (Fractogel HW40S, 3 Trennsäulen je 120 cm Länge, 10 cm Durchmesser) bei 55°C und einer Durchflussgeschwindigkeit von 600 ml/Stunde fraktioniert. Die Difructosedianhydrid-haltigen Fraktionen wurden vereinigt und auf 15% Trockensubstanz aufkonzentriert.

Diese Lösung wurde mit mikrofiltriertem VE-Wasser als Elutionsmittel an einer Umkehrphase (Nucleosil 120-7-C18, 34 cm Länge, inklusive Vorsäule, 2,1 cm Durchmesser) bei einer Durchflussgeschwindigkeit von 5 ml/min bei Raumtemperatur chromatographiert und fraktioniert. Nach wiederholtem Auftragen (0,2 ml pro Trennung) wurden 5 g Trockensubstanz chromatographiert und entsprechend fraktioniert, wobei 1,3 g DFA I mit einer Reinheit von 95 % sowie 1,1 g DFA II mit einer Reinheit von 91 % erhalten wurden.

### Difructosedianhydrid III (DFA III)

Von einer Schrägagar-Kultur wurde eine Abimpfung des Stammes *Arthrobacter ureafaciens* (ATCC 21 124) in einen Schüttelkolben (300 ml) mit einem Nährmedium (pH 7,0), bestehend aus 6 g/l Inulin (Raftiline® St, Fa. Orafti), 2 g/l Dinatriumhydrogenphosphat, 1 g/l Kaliumdihydrogenphosphat, 1 g/l Ammoniumnitrat, 0,5 g/l Magnesiumsulfat, 0,01 g/l Eisensulfat, 0,03 g/l Calciumsulfat und 0,5 g/l Hefeextrakt, überführt. Danach erfolgte eine 24-stündige Inkubation bei 27°C und 120 Upm.

Die durch Zentrifugation erhaltene Biomasse (3 g) wurde mit einer 10%-igen Inulinlösung (Raftiline® St), die mit 0,02 M Phosphatpuffer auf einen pH-Wert von 5,5 eingestellt worden war, auf 300 ml aufgefüllt. Danach erfolgte unter Schütteln eine 4-stündige Inkubation bei 40°C. Nach Beendigung der Reaktion wurde die Biomasse mittels Zentrifugation abgetrennt und der erhaltene Überstand mikrofiltriert (0,45 µm).

Das Filtrat wurde nach Aufkonzentrierung auf 15% Trockensubstanz mittels Gelpermeations-Chromatographie (Fractogel HW40S, 3 Trennsäulen, je 120 cm Länge, 10 cm Durchmesser) bei 55°C und einer Durchflussgeschwindigkeit von 600 ml/Stunde fraktioniert.

Dabei wurden 15 g DFA III mit einer Reinheit von 95% erhalten.

### Fructooligosaccharide (FOS)

60 kg vorzerkleinerte Zichorienwurzeln wurden nach Zugabe von 120 1 Wasser mittels Supratonmaschine in einem kontinuierlichen Verfahren zu einem Brei verarbeitet. Dabei wurde der Brei auf 95°C erhitzt und 30 min bei dieser Temperatur belassen. Nach Abkühlen des Breies auf 56°C wurde der pH-Wert mit 33%-iger H₂SO₄ auf 5,4 eingestellt. Nach Zugabe von 2 Einheiten Endo-Inulinase (SP 168, Novo) pro Gramm Inulin wurde der Ansatz 15 Stunden bei 56°C gerührt. Durch Zudosierung von Kalkmilch wurde der pH-Wert konstant bei 5,4 gehalten.

Die enzymatische Reaktion wurde gestoppt, indem der pH-Wert durch Zugabe von Ca(OH)₂ auf 10,7 erhöht wurde. Nach 30 Minuten wurde der Feststoff mittels Filtrierkammerpresse abgetrennt und nochmals mit 50 1 Wasser gewaschen. Die vereinigten Filtrate wurden anschliessend durch die Zugabe von Kohlensäure neutralisiert.

Das Produkt wurde entweder direkt nach Aufkonzentrierung beziehungsweise anschließender Trocknung verwendet oder wurde zuvor einer Entsalzung und Entfärbung unterzogen. Entsalzung und Entfärbung erfolgten unter Verwendung eines schwach sauren Kationenaustauschers in der H⁺-Form (beispielsweise Lewatit® CNP 80, Bayer AG) und unter Verwendung eines mittelbasischen Anionenaustauschers in der OH-Form (beispielsweise Lewatit® MP 64, Bayer AG). Die Säulen waren hintereinander geschaltet, so dass die Produktlösung zuerst den Kationenaustauscher und dann den Anionenaustauscher passierte. Dieser Schritt wurde bei Raumtemperatur durchgeführt.

Das FOS-haltige Eluat wurde auf 20% Trockensubstanz auf konzentriert und anschliessend sprühgetrocknet. Dabei wurden 7,85 kg Produkt erhalten.

Tabelle 2 zeigt die Zusammensetzung des Produktes nach gelchromatographischer Trennung.

**Tabelle 2 Zusammensetzung der hergestellten Fructooligosaccharide**

| | % Fläche |
|---|---|
| Monosaccharide | 5,7 |
| Disaccharide | 10,3 |
| Trisaccharide. | 20,3 |
| Tetrasaccharide | 20,3 |
| Pentasaccharide | 16,8 |
| Hexasaccharide | 12,6 |
| Heptasaccharide | 7,5 |
| Heptasaccharide | 6,5 |

Bezogen auf die Trockensubstanz, betrug der Gehalt an Glucose, Fructose und Saccharose 3,4%, 2,3% beziehungsweise 6,5%.

### Hydrierte Fructooligosaccharide

1 kg langkettiges Inulin (zum Beispiel HP-Inulin, Orafti) mit einer mittleren Kettenlänge von 25'wurde in 30 1 VE-Wasser eingerührt und durch Erhitzen auf 85°C gelöst. Nach Abkühlen der Lösung auf 54°C und Einstellen des pH-Wertes auf 5,3 durch Zugabe von 1 N NaOH wurden 2.000 Einheiten Endo-Inulinase (Produktbezeichnung "SP 168", (Novo) zugegeben. Der Ansatz wurde 48 Stunden bei 54°C langsam gerührt und anschliessend die Reaktion durch 20-minütiges Erhitzen auf 95°C gestoppt.

Eine HPLC-Änalyse zeigte, dass das Produkt, bezogen auf die Trockensubstanz, zu 83% aus homooligomeren Fructooligosacchariden mit einer DP-Verteilung von 2 - 7 bestand. Der verbliebene Rest bestand aus heterooligomeren Fructooligosacchariden und freier Fructose.

Die auf 40% Trockensubstanz eingedampfte Lösung wurde in einem Laborautoklaven in Gegenwart von Raney-Nickel 18 Stunden mit Wasserstoff bei 150 bar und 80°C hydriert. Die erhaltene Lösung wurde filtriert und mittels Ionenaustauscher gereinigt.

Die gereinigte Lösung kann in flüssiger Form verwendet werden. Sie kann jedoch auch unter Verwendung bekannter Trocknungsverfahren, beispielsweise Sprühtrocknung, in eine trockene Form überführt werden.

### Chitooligosaccharide

25 g Chitin (Primex) mit einer Korngröße von ≥ 0,12 mm wurden mit 100 ml 12 M HCl in einem Dreihalskolben (250 ml) 10 Minuten bei 20°C verrührt. Danach wurde das Chitin einer 110-minütigen Hydrolyse bei 40°C unterworfen. Zur Neutralisation wurde das Reaktionsgemisch in ein 400 ml-Becherglas überführt und auf 5°C gekühlt. Die Neutralisation erfolgte durch langsame Zugabe von eiskalter 50%-iger NaOH-Lösung unter Rühren, wobei die Temperatur kontrolliert wurde (T < 25°C). Feststoffe wurden danach mittels Zentrifugation abgetrennt. Der Rückstand wurde dreimal mit VE-Wasser gewaschen und die vereinigten Überstände filtriert (0,45 µm) und anschließend entsalzt.

Die Entsalzung erfolgte mittels Elektrodialyse (BEL 2, Fa. Berghof ).

Bei einer Leitfähigkeit von 10 mS/cm und einer Spannung von 8 V war die Entsalzung nach 44 Stunden weitgehend abgeschlossen (Endwert: 0,28 mS/cm). Danach wurde die Produktlösung auf 10% Trockensubstanz aufkonzeritriert und anschließend einer Gefriertrocknung unterworfen. Dabei wurden 1.7,5 g Chitooligosaccharide erhalten. Tabelle 3 zeigt die Zusammensetzung des erhaltenen Produktes nach gelchromatographischer Trennung.

**Tabelle 3 Zusammensetzung der hergestellten Chitoologosaccharide**

| | % Fläche |
|---|---|
| DP 1 | 34,8 |
| DP 2 | 18,8 |
| DP 3 | 14,7 |
| DP 4 | 11,1 |
| DP 5 | 8,6 |
| DP 6 | 5,8 |
| >DP 6 | 6,0 |

### Chitosanoligosaccharide

Zunächst wurde ein vollständig deacetyliertes Chitosan hergestellt (A. Domard und M. Rinando, Int. I. Biol. Macromol., 5 (1983), 49-52).

12 g des deacetylierten Chitosans wurden in einem Dreihalskolben (1 Liter) mit 600 ml 12 M HC1 gelöst und 3 Stunden bei 72°C gerührt. Die Reaktion wurde durch Abkühlen auf 4°C gestoppt. Anschließend wurde die Lösung am Rotationsverdampfer bis zur Trockene eingedampft, zweimal in je 500 ml Wasser aufgenommen und wiederum bis zur Trockene eingeengt. Anschließend wurden 300 ml Wasser zugegeben und der pH-Wert der erhaltenen Lösung durch Zugabe von konzentriertem NaOH unter Rühren auf 3 eingestellt.

Die Entsalzung erfolgte mittels Elektrodialyse (BEL 2, Membranen: CMX sowie AMX, Fa. Berghof). Bei einer Leitfähigkeit von > 20 mS/cm und einer Spannung von 8 V war die Entsalzung nach 48 Stunden weitgehend beendet (Endwert: 0,35 mS/cm). Nach Aufkonzentrierung und Gefriertrocknung konnten 9 g Chitosanoligosaccharide erhalten werden. Tabelle 4 zeigt die Zusammensetzung des Produktes nach gelchromatographischer Trennung (Fractogel HW40S, 2 Trennsäulen, 2,6 x 150 cm; Eluent: 0,25 M Ammoniumacetat, pH 4,0).

**Tabelle 4 Zusammensetzung der hergestellten Chitosanoligosaccharide**

| | % Fläche |
|---|---|
| DP 1 | 7,4 |
| DP 2 | 7,4 |
| DP 3 | 6,3 |
| DP 4 | 5,9 |
| DP 5 | 4,7 |
| DP 6 | 3,5 |
| > DP 6 | 64,8 |

### Pektin-Hydrolysat

In 180 1 VE-Wasser wurden 6 kg hochverestertes Citruspektin eingerührt. Nach Zugabe von 200 ml einer Pektinlyase (zum Beispiel Rohapect PTE, Röhm) erfolgte eine 2-stündige Inkubation bei 45 °C und einem pH-Wert von 4,5. Danach wurden weitere 14 kg HV-Citruspektin eingerührt und der pH-Wert wurde mittels 1 N NaOH wiederum auf 4,5 eingestellt. Nach Zugabe von 200 ml Endo-Polygalacturonase (zum Beipiel Pectinase PL, Firma Amano) wurde die Inkubation weitere 17 Stunden unter Rühren fortgeführt. Dann wurden die Enzyme durch 10-minütiges Erhitzen bei 80°C inaktiviert.

Der unlösliche Rückstand wurde durch Zentrifugation entfernt und die klare Lösung wurde auf 45% TS konzentriert und gegebenenfalls getrocknet. Tabelle 5 zeigt die Zusammensetzung des erhaltenen Produktes.

**Tabelle 5 Zusammensetzung des hergestellten Pektin-Hydrolysats**

| | |
|---|---|
| - Kohlenhydrate | |
| DP 1 | 7,0 % |
| Galacturonide davon | 77,4 % |
| ungesättigte Galacturonide | 37% |
| DP2 -DP40 | 78% |
| DP > 40 | <0,1 % |
| Veresterungsgrad | 67,5 % |
| - Salzgehalt | 7,4% |
| - Rohprotein | 1,0% |
| Wassergehalt | 4,1 % |

In einer bevorzugten Ausführungsform wurden getrocknete Orangenschalen oder Citrus-Pellets auf eine Partikelgröße von ca 1-5 mm zerkleinert. 100 g davon wurden in 400 ml Wasser eingerührt, wobei die Partikel aufquollen. Dann wurde konzentrierte Salzsäure (8 g) hinzugefügt. Die Suspension wurde auf 85°C erwärmt und bei dieser Temperatur 1,5 Stunden gerührt. Nach Abkühlung auf 45°C wurde der pH-Wert mit NaOH auf 4,5 angehoben. Nach Zugabe von 0,3 ml einer Pektinlyase (beispielsweise Rohapect PTE, Firma Röhm) erfolgte eine 2-stündige Inkubation. Dann wurden 0,75 ml einer Endopolygalakturonase (beispielsweise Pectinase PL, Firma Amano) hinzugefügt und die Inkubation wurde bei unveränderten Reaktionsbedingungen weitere 3 Stunden fortgeführt. Danach wurden die Enzyme durch Erhitzung auf 95°C inaktiviert. Die erhaltene Suspension wurde aufkonzentriert und an einem Walzentrockner getrocknet.

### Beispiel 2

### Untersuchung zur Stabilität der Saccharide in Magen und Dünndarm

### Stabilität im Magen

Die Stabilität einer Substanz bei der Magen-Passage läßt sich durch Bestimmung der Hydrolysate bei einem pH-Wert von 1,0 beziehungsweise 2,0 darstellen und mit Saccharose als Kontrolle vergleichen. Zur Kontrolle wurde eine 1%-ige Saccharidlösung bei einem pH-Wert von 1,0 (0,1 M HCl) und einem pH-Wert von 2,0 (0,01 M HCl) 3 Stunden bei 37°C inkubiert. Aus den jeweiligen Reaktionsansätzen wurden nach 60, 120 und 180 Minuten Proben entnommen und mittels HPAEC-Verfahren analysiert. Die Ergebnisse sind in der folgenden Tabelle 6 dargestellt (Angabe als Hydrolysate in %).

**Tabelle 6 Stabilität von Sacchariden nach Magen-Passage**

| **Substanz** | **Inkubationszeit (min)** | | | |
|---|---|---|---|---|
| | **pH** | **60** | **120** | **180** |
| Saccharose | 1 | 24 | 55 | 61 |
| | 2 | 1 | 2 | 7 |
| Condensed Palatinose | 1 | < 1 | < 1 | < 1 |
| | 2 | < 1 | < 1 | < 1 |
| DFA I | 1 | < 1 | < 1 | 4 |
| | 2 | < 1 | < 1 | < 1 |
| DFA II | 1 | < 1 | < 1 | 2 |
| | 2 | < 1 | < 1 | < 1 |
| DFA III | 1 | < 1 | < 1 | 3 |
| | 2 | < 1 | < 1 | < 1 |
| Fructooligosaccharide | 1 | 90 | 100 | 100 |
| | 2 | 15 | 30 | 42 |
| Hydrierte Fructooligosaccharide | 1 | 8 | 90 | 100 |
| | 2 | 12 | 25 | 35 |
| Chitooligosaccharide | 1 | < 1 | < 1 | < 1 |
| | 2 | < 1 | < 1 | < 1 |
| Chitosanoligosaccharide | 1 | < 1 | < 1 | < 1 |
| | 2 | < 1 | < 1 | < 1 |
| Pektin-Hydrolysat | 1 | 2 | 3 | 8 |
| | 2 | < 1 | < 1 | < 1 |

Aus dieser Tabelle ist ersichtlich, dass die Saccharide nicht oder nur partiell gespalten werden und somit die Magen-Passage vorzugsweise unbeschadet überstehen.

### Stabilität gegenüber Pankreas-Enzymen

Pankreas-Sekret enthält eine große Anzahl von Hydrolasen, unter anderem auch kohlenhydratspaltende Enzyme wie α-Amylase, welche α-1,4-Glucane (Stärke, Glykogen) vorzugsweise zu Maltose und Maltooligosacchariden spaltet.

Die Untersuchung der Stabilität von Sacchariden gegenüber Pankreas-Enzymen wurde wie folgt durchgeführt.

Die zu untersuchenden Saccharide wurden jeweils in 6 mM NaCl enthaltendem 20 mM Na-Phosphat-Puffer, pH 7,0 (Lösung 1) gelöst, so dass eine 1%-ige Lösung erhalten wurde. Zur Kontrolle wurde eine 1%-ige Stärkelösung (lösliche Stärke nach Zulkowski) in Lösung 1 hergestellt. Außerdem wurde eine 0,2%-ige Pankreatin-Lösung hergestellt, wobei Pankreatin (Fa. Sigma) in Lösung 1 gelöst wurde. Zu 3,0 ml jeder Probenlösung und zu 3,0 ml der Kontrolle wurden 0,1 ml der zuvor hergestellten Pankreatin-Lösung gegeben. Nach einer 210-minütigen Inkubation im Thermomixer (Intervall-Schütteln) bei 37°C wurde die Reaktion durch 15-minütiges Erhitzen auf 95°C beendet. Die stärkehaltige Probe wurde dabei durch 3-stündiges Erhitzen in 1 M HC1 bei 95°C vollständig hydrolysiert. Danach wurden die Proben mittels HPAEC analysiert und die entstandene Glucose gemessen. Die Ergebnisse sind in der folgenden Tabelle 7 gezeigt.

**Tabelle 7 Stabilität von Sacchariden gegenüber Pankreas-Enzymen**

| **Substanz** | **Abbaurate (%)** |
|---|---|
| Stärke | 77 |
| Condensed Palatinose | < 1 |
| DFA I | < 1 |
| DFA II | < 1 |
| DFA III | < 1 |
| Fructooligosaccharide | < 1 |
| Hydrierte Fructooligosaccharide | < 1 |
| Chitooligosaccharide | < 1 |
| Chitosanoligosaccharide | < 1 |
| Pektin-Hydrolysat | < 1 |

Tabelle 7 zeigt, dass die erfindungsgemäß verwendeten Saccharide durch die Pankreas-Enzyme nicht angegriffen werden.

### Spaltbarkeit durch Dünndarm-α-Glucosidasen

Die im Dünndarm vorhandenen, aus der Mucosa stammenden Enzymkomplexe Saccharase/Isomaltase und Glucoamylase/Maltase sorgen in vivo dafür, dass die in den Dünndarm gelangten Disaccharide Maltose und Saccharose und zum Teil auch Maltooligosaccharide zu Monosacchariden gespalten werden und als solche über die Darmwand in den Blutkreislauf gelangen.

Die Prüfung der Stabilität von erfindungsgemäßen Sacchariden gegenüber diesen Enzymen wurde wie folgt durchgeführt.

Zunächst wurden die Enzymkomplexe Saccharase/Isomaltase (SI-Komplex) und Glucoamylase/Maltase (GM-Komplex) aus Schweine-Dünndarm nach dem Verfahren von H. Heymann (Dissertation, Hannover, 1991) isoliert.

Die zu untersuchenden Saccharide wurden in 0,1 M Triethanolamin-Puffer (TRA), pH-Wert 7,0, gelöst, so dass eine 1%-ige Lösung erhalten wurde. Zur Kontrolle wurden von Maltose und Saccharose unter Verwendung des gleichen TRA-Puffers 1%-ige Lösungen hergestellt. Die vorstehend isolierten Mucosa-Enzyme wurden ebenfalls in TRA-Puffer gelöst.

Zu 1,2 ml der auf 37°C temperierten Kohlenhydratlösung wurden 0,7 U (units) des Enzymkomplexes Saccharase/Isomaltase beziehungsweise des Enzymkomplexes Glucoamylase/Maltase zu t = O zugegeben. Nach Mischen wurde jeder Reaktionsansatz bei 37°C inkubiert. Jede Reaktion wurde nach 2 Stunden durch 15-minütiges Erhitzen auf 95°C gestoppt. Die gebildeten Monosaccharide sowie die eingesetzten Testsubstanzen wurden mittels HPAEC quantitativ bestimmt. Die Ergebnisse sind in Tabelle 8 dargestellt.

**Tabelle 8 Stabilität von Sacchariden gegenüber Mucosa-Enzymen**

| | | |
|---|---|---|
| Saccharid | **Hydrolysate (%)** | |
| | SI | GM |
| Saccharose | 98 | - |
| Maltose | 95 | - |
| Maltose | - | 96 |
| Condensed Palatinose | 2 | 1 |
| DFA I | < 1 | < 1 |
| DFA II | < 1 | < 1 |
| DFA III | < 1 | < 1 |
| Fructooligosaccharide | < 1 | < 1 |
| Hydrierte Fructooligosaccharide | < 1 | < 1 |
| Chitooligosaccharide | < 1 | < 1 |
| Chitosanoligosaccharide | < 1 | < 1 |
| Pektin-Hydrolysat | < 1 | < 1 |

Die Ergebnisse zeigen, dass unter den gewählten Bedingungen Saccharose beziehungsweise Maltose durch den SI-Enzymkomplex fast vollständig hydrolysiert wurden. Darüberhinaus wurde Maltose auch durch den GM-Komplex fast vollständig hydrolysiert. Im Gegensatz dazu wurden die erfindungsgemäß zur Behandlung bakterieller Darminfektionen eingesetzten Saccharide durch beide Enzymkomplexe praktisch nicht oder nur geringfügig gespalten.

### Beispiel 3: In vitro-Untersuchung der Fermentation zu SCFA

Die Substanzen Galactomannanoligosaccharide, Difructosedianhydrid II (DFA II) und "condensed palatinose" wurden in vitro hinsichtlich ihrer Fermentation zu kurzkettigen Fettsäuren untersucht. Dazu wurden von fünf Schweinen frische Fäzes entnommen und zu gleichen Teilen zu einer Mischprobe vereinigt. Aus dieser Probe wurde mit Hilfe eines Stomacher-Kneters eine 10%-ige Suspension in anaerobem 50 mM Phophatpuffer, pH 7,0 hergestellt. Jeweils 0,5 ml dieser Suspension wurden in 9,5 ml eines Mediums gegeben, dessen Zusammensetzung in Tabelle 9 gezeigt ist, und unter anaeroben Bedingungen in Hungate-Röhrchen 28 h bei 37 °C inkubiert.

**Tabelle 9 Zusammensetzung des Mediums, das zur Untersuchung der in vitro-SCFA-Fermentation verwendet wurde**

| | |
|---|---|
| Trypticase/Trypton | 1,5g |
| Yeast extract | 1,0g |
| KH₂PO₄ | 0,24 g |
| Na₂HPO₄ | 0,24 g |
| (NA₄)₂SO₄ | 1,24 g |
| NaCl | 0,48 g |
| MgSO₄ x 7H₂0 | 0,10 g |
| CaCl₂ x 2 H₂O | 0,06 g |
| FeSO₄ x 7H₂O | 2mg |
| Resazurin | 1 mg |
| Cystein/HCl | 0,5g |
| Vitaminlösung (nach DSM 141) | 0,5ml |
| Spurenelementelösung (nach DSM 141) | 9,0ml |
| NaHCO₃ | 2,0g |
| Kohlenhydrate (KH) | 5,0g |
| H₂O dest. | ad 1000 ml |

In einem Kontrollexperiment wurde das Medium mit Fäzessuspension ohne Kohlenhydratzusatz inkubiert. Die folgenden Tabellen 10 bis 13 zeigen die Geschwindigkeit der Fermentation der getesteten Kohlenhydrate, den pH-Wert nach Abschluss der in vitro-Fermentation und die gebildeten kurzkettigen Fettsäuren.

**Tabelle 10 In vitro-Fermentation der Kontrolle (ohne Kohlenhydrat)**

| Zeit (h) | 0 | 7 | 14 | 22 | 28 |
|---|---|---|---|---|---|
| Essigsäure (mmol/l) | 0,53 | 8,77 | 10,65 | 11,55 | 11,57 |
| Propionsäure (mmol/l) | 0,28 | 2,08 | 3,04 | 3,34 | 3,61 |
| Buttersäure (mmol/l) | 0,20 | 2,17 | 2,45 | 2,85 | 3,03 |

pH-Wert nach Abschluss der Fermentation: 7,2

**Tabelle 11 In vitro-Fermentation von Galactomannanoligosacchariden**

| Zeit (h) | 0 | 7 | 14 | 22 | 28 |
|---|---|---|---|---|---|
| Restgehalt KH (%) | 100 | 18,6 | 0 | 0 | 0 |
| Essigsäure (mmol/l) | 3,47 | 17,13 | 23,52 | 23,5 | 24,48 |
| Propionsäure (mmol/l) | 0,24 | 9,39 | 13,66 | 14,11 | 14,58 |
| Buttersäure (mmol/l) | 0,17 | 2,93 | 4,77 | 5,95 | 6,43 |

pH-Wert nach Abschluss der Fermentation: 6,6

**Tabelle 12 In vitro-Fermentation von DFA II**

| Zeit (h) | 0 | 7 | 14 | 22 | 28 |
|---|---|---|---|---|---|
| Restgehalt KH (%) | 100 | 94,1 | 84,6 | 58,7 | 27,7 |
| Essigsäure (mmol/l) | 0,85 | 11,67 | 19,12 | 24,87 | 34,18 |
| Propionsäure (mmol/l) | 0,36 | 2,47 | 4,53 | 11,42 | 15,35 |
| Buttersäure (mmol/l) | 0,2 | 2,23 | 3,68 | 5,42 | 8,02 |

pH-Wert nach Abschluss der Fermentation: 5,4

**Tabelle 13 In vitro-Fermentation von "condensed palatinose"**

| Zeit (h) | 0 | 7 | 14 | 22 | 28 |
|---|---|---|---|---|---|
| Restgehalt KH (%) | 100 | 76,2 | 23,9 | 11 | 4,7 |
| Essigsäure (mmol/l) | 1,0 | 14,7 | 27,37 | 30,28 | 32,53 |
| Propionsäure (mmol/l) | 0,45 | 4,2 | 8,82 | 10,53 | 9,74 |
| Buttersäure (mmol/l) | 0,24 | 3,38 | 8,03 | 10,72 | 10,7 |

pH-Wert nach Abschluss der Fermentation: 5,7

### Beispiel 4: Behandlung von Salmonellen-Infektionen beim Schwein

In einem Schweinemastbetrieb wurden zunächst durch Kotanalysen Salmonellenausscheider identifiziert. Aus den als positive Ausscheider festgestellten Tieren wurden 75 Tiere mit einem Gewicht von 65-75 kg ausgewählt, in drei Gruppen zu je 25 Tieren unterteilt und getrennt in gesonderten Ställen untergebracht.

Die erste Gruppe erhielt "condensed palatinose"-haltiges Futter, die zweite Gruppe Lactobionsäure-haltiges Futter und die dritte Gruppe Futter, das hydrierte Fructooligosaccharide enthielt. Die Dosierung der Kohlenhydrate erfolgte so, dass jeweils 0,5 g Kohlenhydrat pro kg Lebendgewicht und pro Tag verfüttert wurden. Täglich wurden Kotproben entnommen und auf Salmonellen geprüft. Außerdem wurde die Gewichtszunahme der Tiere jeder Gruppe mit der von Kontroll-Tieren (Salmonellenausscheider, Futter ohne Zusätze) verglichen.

Bereits nach einer Behandlungsdauer von 7 Tagen konnten in der Gruppe, an die "condensed palatinose" verfüttert worden war, nur noch zwei Salmonellenausscheider festgestellt werden. In der Gruppe, an die hydrierte Fructooligosaccharide verfüttert worden war, wurde lediglich ein Salmonellenausscheider festgestellt, während in der Gruppe, an die Lactobionsäure verfüttert worden war, überhaupt kein Salmonellenausscheider festzustellen war. Bei der Gewichtsentwicklung waren zwischen der Kontrolle und den behandelten Gruppen keine signifikanten Unterschiede feststellbar.

## Patentansprüche

1. Verwendung von mindestens einem Oligogalacturonid-enthaltendem Pektinhydrolysat als Wirkstoff zur Herstellung eines Arzneimittels zur Behandlung von Darminfektionen eines Tieres.

2. Verwendung nach Anspruch 1, wobei es sich bei dem Tier um ein monogastrisches Tier handelt.

3. Verwendung nach Anspruch 1 oder 2, wobei das monogastrische Tier ein Nutztier ist.

4. Verwendung nach einem der Ansprüche 1 bis 3, wobei das Nutztier ein Schwein oder ein Huhn ist.

5. Verwendung nach Anspruch 1 bis 4, wobei der Wirkstoff in einer pharmazeutischen Zusammensetzung verabreicht wird.

6. Verwendung nach Anspruch 5, wobei der Wirkstoff oral verabreicht wird.

7. Verwendung nach Anspruch 6, wobei der Wirkstoff in Form einer Suspension, Tablette, Pille, Kapsel, eines Granulats oder Pulvers verabreicht wird.

8. Verwendung nach einem der Ansprüche 1 bis 4, wobei das verabreichte Oligogalacturonid-enthaltende Pektinhydrolysat als Zusatz in einem Tierfuttermittel, diätetischem Tierfuttermittel oder Trinkwasser enthalten ist.

9. Verwendung nach einem der Ansprüche 1 bis 8, wobei es sich bei der Darminfektion um eine bakterielle Infektion handelt.

10. Verwendung nach Anspruch 9, wobei es sich bei der bakteriellen Darminfektion um eine von einem Mikroorganismus der Gattung *Salmonella, E.coli* oder *Shigella* verursachte Darminfektion handelt.

11. Tierfuttermittel, enthaltend als Zusatz mindestens ein Oligogalacturonid-enthaltendes Pektin-hydrolysat.

12. Tierfuttermittel nach Anspruch 11, das ein diätetisches Tierfuttermittel ist.
